## Europäisches Patentamt

## European Patent Office

## Office européen des brevets

(11) Publication number: **0 127 677**
**B1**

## EUROPEAN PATENT SPECIFICATION

(45) Date of publication of patent specification: **01.08.90**

(21) Application number: **84900246.4**

(22) Date of filing: **07.12.83**

(86) International application number:
**PCT/US83/01921**

(87) International publication number:
**WO 84/02271 21.06.84 Gazette 84/15**

(51) Int. Cl.⁵: **A 61 K 31/07, A 61 K 31/11, A 61 K 31/12, A 61 K 31/59**

(54) **READILY-ABSORPABLE FATTY ACID EMULSIONS.**

(30) Priority: **09.12.82 US 448153**

(43) Date of publication of application:
**12.12.84 Bulletin 84/50**

(45) Publication of the grant of the patent:
**01.08.90 Bulletin 90/31**

(84) Designated Contracting States:
**AT BE CH DE FR GB LI LU NL SE**

(56) References cited:
| | |
|---|---|
| DE-A-2 936 252 | US-A-4 005 196 |
| GB-A-20 754 58 | US-A-4 075 333 |
| US-A-3 067 104 | US-A-4 201 235 |
| US-A-3 089 823 | US-A-4 214 000 |
| US-A-3 138 532 | US-A-4 284 630 |
| US-A-3 143 475 | US-A-4 284 630 |
| US-A-3 253 992 | US-A-4 297 341 |
| US-A-3 773 930 | US-A-4 297 341 |

Chemical Abstracts, Vol 86, no 12, issued 21 March 1977 (Columbus, Ohio, USA), Abs No 86:78627e, Popovici, 'Vitamin A and D stability in solutions'

(73) Proprietor: **Advanced Drug Technology, Inc.**
Fidelity Bank Plaza, Tower III 11590 North Meridian Street Suite 260
Carmel, Indiana 46032 (US)

(72) Inventor: **BILTON, Gerald, L.**
P.O Box 2345
Zionsville, IN 46077 (US)

(74) Representative: **Vossius & Partner**
Siebertstrasse 4 P.O. Box 86 07 67
D-8000 München 86 (DE)

(56) References cited:
Chemical Abstracts, Vol 92, No 10, issued 10 March 1980 (Columbus, Ohio, USA) Abstract No 92:82322t, Cadorniga et al, 'Stability of vitamin A acetate in microcapsules'

Chemical Abstract, Vol 99, No 4, issued 25 July 1983, (Columbus, Ohio, USA), Abstract No. 99:28036j, Asahi 'Stable emulsions or solutions'

Chemical Abstract, Vol 100, No 6, issued 6 February 1984, (Columbus, Ohio, USA), Abstract No 100:39628u, Richter et al., 'Concentrated hydrosols containing lipophilic and hydrophilic vitamins'.

# EP 0 127 677 B1

**Description**

Complex foods are broken down to simple molecules by the process of digestion. The proximal intestine is the major site of absorption for iron, calcium, water-soluble vitamins and fats. Long chain triglycerides are ingested in the diet. Bile acids secreted by the gall bladder emulsify these water-insoluble lipids in the small intestine, thereby greatly increasing the surface area of the molecules and enhancing the activity of pancreatic lipase. In the lipolytic phase, lipase splits the food triglycerides into monoglycerides and fatty acids.

Certain polyunsaturated fatty acids are essential dietary components since they cannot be manufactured by the body. Among the essential fatty acids, linolenic acid, di-homo-gamma-linoleic acid and arachidonic acid have been shown to be necessary for the tissue biosynthesis of the prostaglandins, which perform vital hormone-like activities in the transmission of genetic information in all cells. For example, the blood vessel endothelium is the site of biosynthesis of a prostaglandin known as $PGI_2$, which functions to maintain the integrity of blood vessel walls and thus prevent the adherence or aggregation of platelets which may lead to clotting and other thromboembolic disorders. Other prostaglandins have been shown to be necessary to maintain the optimal tone of bronchial tissue and to regulate blood pressure. Many long-chain unsaturated fatty acids have been shown to be biosynthetic prostaglandin precursors, i.e. arachidonic acid is the direct biosynthetic precursor of $PGI_2$.

The bile acids aggregate the fatty acids into micelles of a diameter of about 2,5 nm which are solubilized in water. In the mucosal phase of the intestine, the lipid contents are absorbed and long chain fatty acids of greater than ten carbon atoms are re-esterified intracellulary and formed into chylomicrons of less than 0.5 micrometers in diameter, by the conjugation with lipoproteins, phospholipid and cholesterol. As chylomicrons, lipids pass through the epithelial cells and basement membrane, eventually finding their way into the central lacteal of the villi of the small intestine. From there, the chylomicrons are secreted into the lymphatic system, passed through the thoracic duct and emptied into the portal venous system.

Any disorder which decreases the ability of the pancreas to excrete lipase results in the malabsorption of fat. Pancreatic exocrine insufficiency results in severe cases of lipid malabsorption. Chronic pancreatis due to alcoholism and cystic fibrosis are the most common causes of this insufficiency. Pancreatic carcinoma may also cause malabsorption if the pancreatic duct is occluded.

A deficiency in bile acids causes a decrease in the activity of lipase. Any disorder which decreases the synthesis, secretion or reabsorption of bile acids can result in fat malabsorption by impairing emulsification and micelle formation. Such disorders include liver disease, intrahepatic cholestatis, bile duct occlusion, bacterial overgrowth in the small intestine and inflammatory or infiltrative diseases of the small intestine.

Such conditions lead to the malabsorption of fat-soluble vitamins, in multiple deficiencies. Vitamin K deficiency causes impaired blood coagulation. Vitamin A deficiency causes night blindness and loss of the integrity of the epitelial membranes and connective tissue. Vitamin D deficiency along with malabsorption of protein and calcium results in osteomalacia.

Even when the concentration of bile acids is not pathologically decreased, it may be desirable to enhance the lymphatic absorption of fatty acids and fat-soluble vitamins and other biologically-active agents. For instance, orally-ingested 13-cis-retinoic acid, a Vitamin A analog, has been shown to accomplish the prolonged remission of cystic and conglobule acne. Increased serum vitamin A has alleviated conditions such as type II hyperlipoproteinaemia, postilectomy diarrhoea, and osteomalacia. *See* D. L. Barnard, *Gut, 14,* 316 (1973). Vitamin A and Vitamin E and their analogs may play a role in the protection of cells from carcinogens. *See* M. B. Sporn, *et al,* Federation Proc., *35,* 1332 (1976). Fatty acids have been shown to suppress serum cholesterol and have been asserted to be useful for the treatment of thromboembolic disorders. Vitamin K apparently acts to enhance the action of cytotoxic agents useful in chemotherapy.

It is therefore an object of the present invention to provide a composition which will enhance the lymphatic absorption of naturally-occurring essential fatty acids.

It is another object of the present invention to provide a means for enhancing the intestinal absorption of fat-soluble vitamins, vitamin analogs and other biologically-active agents.

Still other objects and advantages of the present invention will be apparent from the description and appended claims.

The objects of the present invention are accomplished by the oral administration to a human or other mammal of oil-in-water emulsions which are formulated so as to contain effective amounts of essential fatty acids, such as linoleic, linolenic and arachidonic acid, in a physical form which is readily absorbed into the lymphatic system and thus into the blood stream. The emulsions of the present invention are also useful to enhance the absorption of vitamins, particularly the oil-soluble vitamins and their analogs. Other oil-phase soluble pharmaceuticals may also be effectively delivered to the blood stream via the present compositions. The compositions of the present invention also greatly reduce, or eliminate the need for the fat emulsification by bile acids, since the emulsions are prepared so that the oil-phase droplets are of a diameter equivalent to or less than the average diameter of the naturally-formed lipoprotein chylomicrons, i.e. up to 0.5 micrometers.

The compositions of the present invention are oil-in-water emulsions which are prepared by

2

emulsifying a vegetable or animal oil rich in polyunsaturated, long-chain fatty acids with an emulsifying agent in the presence of one or more polyhydric alcohol stabilizers, and antibacterial or antifungal preservatives. Oil-soluble nutrients, such as the naturally-occurring amino acids, vitamins and their analogs may also be included in the emulsions, as well as minor amounts of coloring and flavoring. Buffers may also be included when necessary.

The animal or vegetable oils useful in the emulsions of the present invention include those which contain a high proportion of unsaturated $C_{12}$—$C_{18}$ fatty acids to $C_{14}$—$C_{18}$ saturated fatty acids, since it is the polyunsaturated fatty acids which have been implicated in the *in vivo* biosynthesis of biologically active prostaglandins. The preferred oils possess an unsaturated fatty acid to saturated fatty acid ratio of from 10:1 to 5:1. Preferred vegetable oils include primrose oil, safflower oil, sunflower oil, sesame oil and cottonseed oil. Of the vegetable oils sesame oil is especially preferred, since it can be readily stabilized by natural emulsifying agents at an effective droplet size for rapid gut absorption. Fish oil is the preferred animal oil for the formation of the present emulsions, since it contains large amounts of eicosapentaenoic acid (EPA), a PGI precursor.

The vegetable or animal oil or mixture of oils will make up the major proportion of the oil phase of the present emulsions, and preferably will comprise 5—45% by weight of the entire emulsion, most preferably 10—30%.

The oils are suspended in water to form the emulsions of the present invention by means of one or more emulsifying agents. Any non-toxic, pharmacologically-acceptable emulsifying agent may be employed in emulsions, such as those discussed in *Remington's Pharmaceutical Sciences*, A. Osol, ed., Mack Pub. Co., Boston, Mass. (16th ed., 1980) at pages 310—321 and 1243—1248. Preferred emulsifying agents are the natural surfactants, which function by coating oil-phase droplets with a multimolecular film so as to accomplish the necessary dispersion. Multimolecular film-forming agents are preferred due to their ability to form strong, coherent films which render them highly resistant to coalescence, even in the absence of a well-developed surface potential. Especially preferred natural agents include acacia (HLB ca. 8.0), gelatin (HLB ca. 10.5), tragacanth (HLB ca. 13.2), carrageenan, chrondus and pectin and mixtures thereof. Alternatively, or in combination with the foregoing, other conventional emulsifying agents can be employed—such as polyhydric fatty acid esters. In accordance with known techniques the HLB of the emulsifier, or emulsifier combination is adapted to be compatible with the requirements of the oil phase. Preferably the emulsifying agent or agents will comprise from 5—40% by weight of the emulsion, preferably 10—30%, wherein the sum of the oil and emulsifier is about equal to or less than the amount of water in the emulsion.

Stabilizing agents are also used in the emulsions of the present invention. They function to increase the viscosity of the aqueous phase, thus stabilizing the emulsion, and to prevent the precipitation of the emulsifying agents by dissolved metal salts which may be present. Useful stabilizing agents may be selected from any of those known to be compatible with the emulsifying agents used, and include fatty alcohols, methyl cellulose and hydroxypropyl celluloses. An especially preferred class of stabilizing agents is the $C_2$—$C_8$ polyhydric alcohols, i.e. glycerin, sorbitol, and mixtures thereof. These nontoxic agents are easily absorbed in the intestinal tract and may aid the absorption of other components of the emulsion. The stabilizing agents will comprise from 1—20% by weight of the emulsions of the present invention, preferably from 5 to 10%.

The emulsions of the present invention will include a water-soluble buffer system, i.e. an organic acid and acid salt pair, in an amount effective to stabilize the pH of the emulsion so as to protect intestinal flora and to offset the effect of added nutrient acids. A preferred organic buffer system for use in the compositions of the present invention is a 3:1 ratio of citric acid-sodium citrate.

When present, the buffering chemicals will make up 0.25—2.5% by weight of the emulsion.

The emulsions of the present invention will also include minor but effective amounts of antibacterial and antifungal agents which act to maintain the emulsion in a sterile conditon. Preferred antibacterial agents are the ($C_1$—$C_4$) lower alkyl 4-hydroxybenzoate esters such as methyl and propyl 4-hydroxy benzoate and mixtures thereof. Sodium benzoate is a preferred antifungal agent. These antimicrobial agents will preferably make up 0.05—1% by weight of the emulsion.

The oil phase of the present emulsions may also be used to solubilize and deliver dietetically- and therapeutically-effective amounts of oil-soluble nutrients and pharmaceuticals. One preferred class of nutrients is the fat-soluble vitamins which comprise vitamins A, D, E and K and their biologically-active derivatives and analogs. These vitamins may be incorporated into the present emulsions in amounts which will vary in accord with accepted medical practice, both for the alleviation of dietary deficiencies and for therapeutic applications, such as those discussed hereinabove. The vitamin emulsions of the present invention contain a digestible vegetable oil carrier for said vitamin comprising long chain unsaturated fatty acids compatible with and in an amount greater than said vitamin, wherein the combined amount of vitamin and carrier is at least ten percent by weight less than the amount of water. Fat soluble vitamins useful in the practice of the present invention include vitamin A (retinol, *trans*-retinoic acid, 13-*cis*-retinoic acid, retinol-acetate, retinol palmitate). The present emulsions may be formulated so as to deliver from 2000—one million USP units of these compounds per dose of emulsion (i.e. about one-half teaspoon).

Another useful fat-soluble vitamin is vitamin E and its derivatives, i.e. *d* or *dl*-alpha-tocopherol, the alpha-tocopheryl acetates and succinates. The present emulsions may be formulated so as to comprise 20—10,000 USP units ("IU units") of these compounds per dose of emulsion, preferably 250—600 USP units per one-half teaspoon dose. Vitamin E compounds such as alpha-tocopheryl acetate also function as antioxidants to stabilize the unsaturated fatty acids present in the animal or vegetable oil component of the emulsion, and also act to stabilize any vitamin A present against oxidation. Additional antioxidants, such as minor amounts (i.e. 0.025—0.5%) of ascorbyl palmitate, may also supplement the antioxidant function of vitamin E or fulfill this function in its entirety. Vitamin D1, 2, 3 and their fat-soluble metabolites such as 25-hydroxy cholecalciferol and 1,25-dihydroxycholecalciferol may also be readily incorporated in the present emulsions. When the emulsions are adapted as dietary supplements for normal individuals, a maximum dose of 50—500 USP units per dose of emulsion is sufficient. The emulsions may be made more highly concentrated to treat specific pathological conditions, i.e. osreomalacia, hypoparathyroidism or hypocalcemic tetany, in accord with accepted medical practice.

Vitamin K1, K2 and their fat-soluble derivatives such as menadione and phytonadione are also preferred vitamins for incorporation into the emulsions of the present invention which are formulated to contain 1—50 mg per dose. More concentrated formulations may be prepared and administered if necessary to treat enteric diseases in which vitamin K absorption is inhibited, such as sprue, regional enteritis, enterocolitis and dysentery.

For a general discussion of the oil (fat)-soluble vitamins, including their analogs, utilities and dosages, see Remington's Pharmaceutical Sciences, at pages 948—955.

The emulsions of the present invention may also include minor but effective amounts of one or more amino acids. Especially preferred are those amino acids which have immunostimulatory activity, i.e. l-lysine, l-arginine or mixtures thereof. Preferably, the amino acids will be present in an amount sufficient to provide 5—15 mg per dose of emulsion, or in an amount equal to 0.25—2% by weight of the emulsion.

The emulsions of the present invention will also optionally comprise a minor but effective amount of coloring and flavoring. Suitable flavoring agents are described in Remington's Pharmaceutical Sciences at pages 1229—1239.

Accordingly, preferred emulsions formulated in accord with the present invention would comprise 20—50% of an oil phase incorporating 10—30% of unsaturated fatty acid-containing vegetable or animal oil, 10—30% of an emulsifying agent, 1—20% of one or more stabilizing agents dispersed to a particle size in the chylomicron range in 30—80%, preferably 40—55% of water incorporating 0.1—2.0% of one or more antimicrobial preservatives and optionally incorporating minor but effective amounts of amino acids, antioxidants, buffering agents and flavorings, wherein water constitutes the continuous phase (O/W). The amount of the emulsifier is similar to that of the oil, and they are preferably within twenty percent of each other by weight, and are soluble in each other. The combined amount of oil and emulsifier is close that of the water and usually from one to twenty percent less than the weight of the water. In the preferred embodiments of the present invention, the emulsions will comprise a biologically-effective amount of one or more oil-soluble vitamins or their derivatives, such as vitamins A, D, E or K.

The utility of the present stabilized oil-in-water emulsions is not limited to the effective lymphatic absorption of fat-soluble vitamins, but extends to the delivery of a wide variety of fat-soluble compounds of pharmaceutical interest, including hormones (i.e. steroids), prostaglandins, gastrointestinal drugs, cardiovascular drugs, respiratory drugs, sympathomimetic drugs, enzymes, analgesics, and the pure extracts of edible oils, such as gossypol, eicosapentaenoic acids and dihomolinoeic acids. Emulsification can be achieved by several procedures which use a high shear force to obtain small particle sizes. High shear force relates to the applied forces which cause two contiguous parts of a body to slide relative to each other in a direction parallel to their plane of contact.

High shear force may be achieved by use of a pressure homogenizer. Such pressure homogenizers as, for example, the Gaulin Homogenizer, function by passing a product, under pressure, through a restricted aperture whereupon an instant pressure drop to less than atmospheric pressure occurs. This causes both a shearing action and cavitation bubbles. The product then strikes an impact ring at velocities up to 17,4 km (57,000 feet) per minute, further shattering the particles by both impact and implosion of the cavitation bubbles.

In another procedure, a technique involving high-speed mixing (i.e. from 3,000 to 12,000 rpm) coupled with a high shear force is used. In this procedure, the effective shear force is dependent on the solids content and the viscosity of the medium being mixed, the speed of mixing, and the geometry of the mixer and the mixing vessel. A type of mixer which achieves this dual function of high speed mixing and shear force is, for example, one employing a single shaft mixer with two separated, serrated circular horizontal shear plates set between two inverted feed cones on a single shaft. Using a mixer, both high speed mixing and high shear force are rapidly achieved.

Various propeller and turbine agitation systems are commerically available to carry out the emulsification.

The general procedure is to form a first mixture comprising mainly the fatty or oily components and emulsifier. A second mixture is formed comprising mainly the water and water-soluble ingredients. These two mixtures are then combined and subjected to high shear agitation under conditions adapted to provide very small particles, preferably of chylomicron size and smaller.

The emulsions of the present invention in a preferred procedure are prepared by first forming a homogeneous mixture of the vegetable and/or animal oils with the oil-soluble pharmaceuticals, oily

4

antioxidants and amino acids by stirring the ingredients together in a standard slurry-type dispersal mixer with rotor-blade circulating element for 20—30 minutes. The emulsifying agent is then added to the stirred, oily pre-emulsion and stirring continued for 30—60 minutes until a homogeneous paste results. In a separate mixer, a solution of 70—85% of the stabilizer is combined with the antibacterial agents and heated via an immersion heater to 70—80°C until dissolution is complete. A 30—40°C solution of the remainder of the stabilizer component in the water containing the flavoring and antifungal agent is then added to the stabilizer-antibacterial solution and vigorous mixing continued for 1—2 hours. This solution is added to the stabilized oil phase with slow steady stirring over 10—15 min. A concentrated aqueous solution of the buffering chemicals is then added.

To achieve the necessary fine droplet size, the emulsion is then passed three times through a high pressure homogenizer at $27,5 \times 10^6$ to $41,2 \times 10^6$ Pa (4000—6000 Psi) in a continuous manner. A 25 94,6 l (gallon) batch of emulsion can be finished in about 3—4 hours.

The finished emulsion is ready for packaging in standard liquid-dispensing containers. Alternatively, the emulsion may be encapsulated in gelatin capsules or suppositories suitable for oral, buccal, anal or vaginal administration.

The invention will be further described by reference to the following detailed examples.

Example I—Vitamin A/E emulsion

A stable oil-in-water emulsion containing Vitamins A and E in the oil phase was prepared by the following procedure.

TABLE I

| Component | Component wt. (grams) |
|---|---|
| Part A | |
| Vitamin A palmitate | 616 |
| Ascorbyl palmitate | 19 |
| Vitamin E acetate | 19 |
| Sesame oil (refined) | 21,610 |
| l-Lysine | 500 |
| l-Arginine | 500 |
| | |
| Part B | |
| Gum acacia (spray-dried, vacuum gassed) | 22,800 |
| | |
| Part C | |
| Glycerin | 6,650 |
| Methyl 4-hydroxy benzoate | 152 |
| Propyl 4-hydroxybenzoate | 38 |
| | |
| Part D | |
| Deionized water | 39,360 |
| Sorbitol (70% in water) | 2,965 |
| Cherry extract | 170 |
| Sodium benzoate | 95 |
| | |
| Part E | |
| Citric acid | 380 |
| Sodium citrate | 125 |
| Water | 900 |
| | |
| Total | 95,899 |

The ingredients of Part A were combined and stirred in a 114 l (30 gal.) Lightnin vertical tank, gear-reduced mixer equipped with an 20,3 cm (8 inch) blade for 25 min. The acacia gum was added with continued stirring for 45 min. until a homogeneous paste results. The ingredients of Part C were combined and mixed separately at 75°C until they had dissolved. The Part D ingredients were combined, heated to 35°C and the Part C ingredients added with vigorous stirring over 1.5 hours. The resultant stabilizer mixture (Part C plus Part D) was added to the oily pre-emulsion (Part A plus Part B) over 10 minutes while maintaining the stirring at as slow a rate as possible. The ingredients of Part E were combined and the solution added to the stirred mixture with a small amount of water to bring the total batch up to 94.6 l (twenty-five gallons). The emulsion was then homogenized to the desired droplet size by three continuous passes through a Gifford-Wood high pressure homogenizer at $34,3 \times 10^6$ Pa (5000 psi) (Gifford Wood, Inc.,

Hudson, N.Y.), at the rate of one full pass per hour. The final creamy emulsion was the consistency of runny toothpaste, and was very stable. Upon storage over an extended period of time the components did not separate.

When a few drops of the emulsion were stirred into a 1000-fold excess of water, a homogenous, opaque suspension formed which did not break or separate when allowed to stand for at least a week at 25°C. The emulsion delivered 25,000 USP units of Vitamin A and 400 USP units of Vitamin E per 4.0 ml dose (one-half teaspoon).

Example 2

A Vitamin E emulsion was prepared by the procedure of Example 1, omitting the Vitamin A Palmitate. An emulsion similar to that of Ex. 1 was observed.

Example 3

A Vitamin A, D3 and E emulsion was obtained by following the procedure of Example 1, but incorporating 5.5 g of Vitamin D3 into Part A. An emulsion similar to that obtained in Ex. 1 was observed.

The emulsion delivered 25,000 USP units of Vitamin A, 400 USP units of Vitamin E and 400 USP units of Vitamin D3 per 4.0 ml dose.

Example 4—Absorption screening study

A screening study was conducted to compare the intestinal absorption of the emulsion of Ex. 3 with that of a standard-type, over-the-counter cod-liver oil preparation containing Vitamins A, D and E, which was dosed with water to prepare an emulsion containing the same proportional USP units of the three vitamins as the emulsion of Ex. 3. Two rats were dosed with approximately 2.5 ml of the Ex. 3 emulsion and two were dosed with 2.5 ml of the cod-liver oil emulsion. Two of the rats were sacrificed 30 min. after administration of the emulsion, and two were sacrificed 2.0 hours after dosing. The rats were sacrificed by exsanguination after carbon dioxide suffocation. Two control animals which had been treated with water and cod-liver oil were also sacrificed and assayed. Cross-sections of the duodenum were taken approximately 1.0 cm from the pylorus, then immediately processed for microscopic examination by sectioning on a freezing microtome.

The frozen sections were stained with Oil Red O and examined microscopically for the presence of fat within the duodenal structure. The amount of Oil Red O staining material, reflecting the amount of absorbed fat, was graded subjectively on a scale of 0, 1+, 2+, 3+ and 4+. The results are summarized in Table II.

TABLE II. Absorption screening study in rats

(Article, grade, and observation)
Control distilled water
Grade 0

No fat globules are recognized within the duodenal structures. Oil Red O staining material is present within the lumen.

Cod liver oil (undosed)
Grade 1+

Occasional Oil Red O staining material is present in the epithelial cell cytoplasms and in interstitial-lacteal areas of the villi.

Dosed cod liver oil—30 minutes
Grade 2+

Oil Red O staining globules are present in both the epithelial cell cytoplasm and in the interstitial-lacteal areas. Size of the globules varies considerably, and there is marked variability in the amount (numbers) of globules within the different villi.

Ex. 3 Emulsion—30 minutes
Grade 2+

Very small Oil Red O stain globules are finely dispersed within the epithelial cytoplasm and a less number of variable size globules are located in the interstitial-lacteal areas.

Dosed cod liver oil—2 hours
Grade 2+

Primarily, Oil Red O staining globules are present in the epithelial cell cytoplasms with lesser numbers in the interstitial-lacteal areas.

Ex. 3 emulsion—2 hours
Grade 4+

Oil Red O staining globules are very numerous within the epithelial cytoplasm, diffusely throughout the villi, and numerous globules are also present within the interstitial-lacteal areas of the villi.

The results outlined on Table II show that the emulsion of Ex. 3 is more evenly and rapidly absorbed into the interstitial-lacteal areas of the villi of the small intestine of the rat than is an oil-in-water emulsion prepared in a standard manner (i.e. by mixing cod liver oil into water in the presence of additional vitamins). A visual examination of the stained sections indicates that absorption in accordance with the present invention was about 100% greater than that with the conventional formulations after two hours. It is expected that the emulsions of Exs. 1—2 as well as emulsions comprising other fatty oil-soluble pharmaceuticals will behave similarly in this test and that this test protocol demonstrates the high efficiency of the absorption of oils and oil-soluble pharmaceutical agents in the mammalian lower digestive tract when they are emulsified to produce the compositions of the present invention.

Therapeutic products have been frequently used in the clinical administration of a drug or nutrient together with a suitable oil. It has been postulated that an aqueous emulsion of a water soluble drug may have an enhanced lymphatic directivity due to the presence of an oil. In connection with this mode of adminstration, due to the normally larger size of the drug particles in an oil emulsion, the drug is maintained in the capillary vessels and gradually permeates into the surrounding tissues over a longer period of time. However, in contrast to prior procedures, the present invention facilitates a rapid and high level of absorption of therapeutic agents.

Accordingly the technique of forming emulsions set forth in this specification is applicable to a wide range of therapeutic agents which are oil-soluble and suitable for emulsification. Even water-soluble drugs can be adapted for use in the present system by coupling them with lipid materials (i.e. referred to as an oil-compatible therapeutic) to make them soluble in the oil carrier.

This invention is particularly applicable for therapeutic agents which are used to treat malabsorption problems, skin disorders (e.g. herpes, acne, psoriasis), prostaglandin-mediated conditions (e.g. thromboembolic, gastrosecretory, and pulmonary disorders), hemapoietic and hematogenic disorders such as anemias, immunodeficient and autoimmune conditions, and diseases of the digestive system because of the rapid and improved absorption which is provided.

## Claims

1. A stable oil-in-water emulsion comprising:
   (a) droplets of one or more long-chain polyunsaturated fatty acid-containing animal or vegetable oils having a diameter of less than or equal to 0.5 micrometers;
   (b) water;
   (c) one or more emulsifying agents;
   (d) one or more stabilizing agents; and
   (e) one or more antimicrobial agents.
2. The emulsion of claim 1 further comprising a pH-stabilizing amount of an organic buffer pair.
3. The emulsion of claim 2 wherein the buffer pair is citric acid-sodium citrate.
4. The emulsion of claim 1 further comprising one or more fat-soluble biologically-active agents.
5. The emulsion of claim 4 wherein the agent comprises a fat-soluble vitamin.
6. The emulsion of claim 5 wherein the vitamin is a nutritionally effective amount of vitamin A, E, D, or K or a mixture thereof.
7. The emulsion of claim 5 further comprising 0.25—2% by weight of one or more amino acids.
8. The emulsion of claim 7 wherein the amino acid is L-lysine, L-arginine or a mixture thereof.
9. The emulsion of claim 5 wherein the oil is sesame oil, safflower oil, primrose oil, sunflower oil, fish oil, cottonseed oil or a mixture thereof.
10. The emulsion of claim 9 wherein the emulsifying agent is effective to suspend the droplets by means of a multimolecular film.
11. The emulsion of claim 5 wherein the emulsifying agent is acacia, gelatin, tragacanth, chondrus, pectin or a mixture thereof.
12. The emulsion of claim 5 wherein the stabilizing agent is a $C_2$—$C_8$ polyhydric alcohol.
13. The emulsion of claim 12 wherein the stabilizing agent is a mixture of glycerin and sorbitol.
14. The emulsion of claim 4 wherein the agent is a prostaglandin, a long-chain unsaturated fatty acid prostaglandin precursor, gossypol or 5-azacytidine.
15. The emulsion of claim 5 further comprising 0.025—0.5% by weight of an antioxidant.
16. A stable vitamin emulsion in water adapted to be rapidly absorbed through cell walls and containing oil phase droplets less than or equal to 0.5 micrometers in diameter comprising:
    (a) 30—80% by weight water;
    (b) an oil soluble vitamin which is vitamin A, D, E, or K, a pharmacologically stable derivative thereof, or a mixture thereof, in a therapeutically effective amount;
    (c) a digestible vegetable oil carrier for said vitamin comprising long-chain unsaturated fatty acids compatible with, and in an amount greater than said vitamin, wherein the combined amount of vitamin and carrier is at least ten percent by weight less than the amount of water; and
    (d) a digestible emulsifier in an amount corresponding to 5—40% of the weight of the emulsion.
17. The product of claim 16 wherein a mixture of said vitamin and carrier comprises emulsified particles in said water having a particle size of less than or equal to 0.5 micrometers in diameter.

18. The product of claim 17 comprising an antimicrobial agent, a buffering agent, and an emulsion stabilizer.

19. The product of claim 18 comprising one or more amino acids.

20. The product of claim 19 comprising a fatty acid derivative of ascorbic acid.

21. The product of claim 19 wherein said oil is sesame or primrose oil;
said emulsifier is gum acacia;
said antimicrobial agent is an alkyl paraben and benzoate;
said buffering agent is a citrate with citric acid;
said emulsion stabilizer is glycerine and sorbitol; and
said amino acids are lysine and arginine.

22. The product of claim 21 comprising:
40—50% by weight water;
10—30% by weight oil;
10—30% by weight emulsifier;
0.05—1.0% by weight antimicrobial agent;
0.25—2.5% by weight buffering agent;
5—10% by weight emulsion stabilizer; and
0.25—2% by weight amino acids.

23. A method of forming a stable oil-in-water emulsion according to any one of claims 1 to 15 which comprises forming an oil phase mixture of an oil-soluble therapeutic, an oil carrier, and an emulsifier; forming a water phase mixture of water, an emulsion stabilizer and an antimicrobial agent; combining and mixing said oil phase and water phase; and subjecting said mixture to a high shear force sufficient to form emulsified particles having a size of less than or equal to 0.5 micrometers in diameter.

24. The method of claim 23 comprising:
(a) forming a homogeneous oil-phase mixture of one or more edible, polyunsaturated fat-containing animal or vegetable oils, one or more fat-soluble vitamins, one or more amino acids, and one or more antioxidants;
(b) adding an emulsifying agent to the stirred mixture so as to form a homogeneous paste;
(c) adding an aqueous solution of one or more polyhydric alcohol stabilizing agents and one or more antimicrobial agents to said stirred paste;
(d) adding a concentrated aqueous solution of organic buffering agents to said paste with slow stirring to form a buffered mixture; and
(e) passing the buffered mixture through a high-pressure homogenizer at $27.5 \times 10^6 - 41.2 \times 10^6$ Pa (4000 to 6000 psi); whereby the emulsified oils and vitamins possess a droplet size of less than or equal to 0.5 micrometers in diameter.

## Patentansprüche

1. Stabile Öl-in-Wasser-Emulsion, umfassend:
(a) Tröpfchen von einem oder mehreren langkettige, mehrfach ungesättigte Fettsäuren enthaltenden tierischen oder pflanzlichen Ölen mit einem Durchmesser von höchstens 0,5 µm;
(b) Wasser;
(c) ein oder mehrere emulgierende Mittel;
(d) ein oder mehrere stabilisierende Mittel; und
(e) ein oder mehrere antimikrobielle Mittel.

2. Emulsion nach Anspruch 1, umfassend ferner eine pH-stabilisierende Menge eines organischen Pufferpaares.

3. Emulsion nach Anspruch 2, wobei das Pufferpaar Citronensäure-Natriumcitrat ist.

4. Emulsion nach Anspruch 1, umfassend ferner ein oder mehrere fettlösliche, biologisch aktive Stoffe.

5. Emulsion nach Anspruch 4, wobei der Stoff ein fettlösliches Vitamin umfaßt.

6. Emulsion nach Anspruch 5, wobei das Vitamin eine unter Ernährungsgesichtspunkten wirksame Menge von Vitamin A, E, D oder K oder ein Gemisch davon ist.

7. Emulsion nach Anspruch 5, umfassend ferner 0,25 bis 2 Gew.-% einer oder mehrerer Aminosäuren.

8. Emulsion nach Anspruch 7, in der die Aminosäure L-Lysin, L-Arginin oder ein Gemisch davon ist.

9. Emulsion nach Anspruch 5, in der das Öl Sesamöl, Safranöl, Primelöl, Sonnenblumenöl, Fischöl, Baumwollsamenöl oder ein Gemisch davon ist.

10. Emulsion nach Anspruch 9, wobei das emulgierende Mittel zur Suspendierung der Tröpfchen mittels eines multimolekularen Filmes wirksam ist.

11. Emulsion nach Anspruch 5, wobei das emulgierende Mittel Akazia, Gelatine, Tragacanth, Chondrus, Pectin oder ein Gemisch davon ist.

12. Emulsion nach Anspruch 5, wobei das stabilisierende Mittel ein $C_2-C_8$-mehrwertiger Alkohol ist.

13. Emulsion nach Anspruch 12, wobei das stabilisierende Mittel ein Gemisch von Glycerin und Sorbit ist.

8

14. Emulsion nach Anspruch 4, wobei der Stoff ein Prostaglandin, eine langkettige, ungesättigte Fettsäure als Prostaglandin-Vorstufe, Gossypol oder 5-Azacytidin ist.

15. Emulsion nach Anspruch 5, umfassend ferner 0,025 bis 0,5 Gew.-% eines Antioxidanz.

16. Stabile Vitamin-Emulsion in Wasser, angepaßt zur raschen Absorbierung durch Zellwände und enthaltend Ölphasen-Tröpfchen mit einem Durchmesser von höchstens 0,5 µm, umfassend

(a) 30 bis 80 Gew.-% Wasser;

(b) ein öllösliches Vitamin, welches Vitamin A, D, E oder K, ein pharmakologisch stabiles Derivat davon oder ein Gemisch davon ist, in einer therapeutisch wirksamen Menge;

(c) ein verdauliches pflanzliches Öl als Träger für das Vitamin, umfassend langkettige ungesättigte Fettsäuren, die mit dem Vitamin verträglich sind und in einer größeren Menge als das Vitamin vorliegen, wobei die vereinigte Menge von Vitamin und Träger mindestens 10 Gew.-% geringer als die Menge Wasser ist; und

(d) einen verdaubaren Emulgator in einer Menge, die 5 bis 40 Gew.-% der Emulsion entspricht.

17. Produkt nach Anspruch 16, wobei ein Gemisch des Vitamins und des Trägers emulgierte Teilchen in dem Wasser umfaßt, die eine Teilchengröße von höchstens 0,5 µm im Durchmesser aufweisen.

18. Produkt nach Anspruch 17, umfassend einen antimikrobiellen Stoff, einen Puffer und einen Emulsionsstabilisator.

19. Produkt nach Anspruch 18, umfassend eine oder mehrere Aminosäuren.

20. Produkt nach Anspruch 19, umfassend ein Fettsäurederivat von Ascorbinsäure.

21. Produkt nach Anspruch 19, wobei das Öl Sesam- oder Primelöl ist;

der Emulgator Akaziagummi ist;

der antimikrobielle Stoff ein Alkylparaben und Benzoat ist;

der Puffer ein Citrat mit Citronensäure ist;

der Emulsionsstabilisator Glycerin und Sorbit ist; und

die Aminosäuren Lysin und Arginin sind.

22. Produkt nach Anspruch 21, umfassend:

40 bis 50 Gew.-% Wasser;

10 bis 30 Gew.-% Öl;

10 bis 30 Gew.-% Emulgator;

0,05 bis 1,0 Gew.-% antimikrobieller Stoff;

0,25 bis 2,5 Gew.-% Puffer;

5 bis 10 Gew.-% Emulsionsstabilisator; und

0,25 bis 2 Gew.-% Aminosäuren.

23. Verfahren zur Erzeugung einer stabilen Öl-in-Wasser-Emulsion nach einem der Ansprüche 1 bis 15, umfassend die Erzeugung eines Ölphasengemisches aus einem öllöslichen therapeutisch wirksamen Stoff, einem Öl-Träger und einem Emulgator, Erzeugung eines Wasserphasen-Gemisches aus Wasser, einem Emulsionsstabilisator und einem antimikrobiellen Stoff, Vereinigen und Mischen der Ölphase und der Wasserphase und Ausüben einer hohen Scherkraft auf das Gemisch, die zur Erzeugung von Emulsionsteilchen mit einer Größe von höchstens 0,5 µm im Durchmesser ausreicht.

24. Verfahren nach Anspruch 23, umfassend:

(a) Erzeugung eines homogenen Ölphasen-Gemisches aus einem oder mehreren eßbaren, mehrfach ungesättigte Fette enthaltenden tierischen oder pflanzlichen Ölen, einem oder mehreren fettlöslichen Vitaminen, einer oder mehreren Aminosäuren und einem oder mehreren Antioxidantien;

(b) Zugabe eines emulgierenden Mittels zu dem Gemisch unter Rühren zur Erzeugung einer homogenen Paste;

(c) Zugabe einer wäßrigen Lösung von einem oder mehreren mehrwertigen Alkoholen als stabilisierenden Mitteln und einem oder mehreren antimikrobiellen Mitteln zu der Paste unter Rühren;

(d) Zugabe einer konzentrierten wäßrigen Lösung von organischen Puffern zu der Paste unter langsamem Rühren zur Erzeugung eines gepufferten Gemisches; und

(e) Hindurchführung des gepufferten Gemisches durch einen Hochdruck-Homogenisator bei $27,5 \times 10^6$ bis $41,2 \times 10^6$ Pa (4000 bis 6000 psi), wobei die emulgierten Öle und Vitamine eine Tröpfchengröße von höchstens 0,5 µm im Durchmesser aufweisen.

**Revendications**

1. Emulsion d'huile dans l'eau stable comprenant:

(a) des gouttelettes d'une ou de plusieurs huiles animales ou végétales contenant des acides gras polyinsaturés à longue chaîne, ayant un diamètre inférieur ou égal à 0,5 micromètre;

(b) de l'eau;

(c) un ou plusieurs agents émulsifiants;

(d) un ou plusieurs agents stabilisants; et

(e) un ou plusieurs agents antimicrobiens.

2. Emulsion suivant la revendication 1, caractérisée en ce qu'elle comprend en outre une quantité stabilisatrice du pH d'une paire de tampons organiques.

3. Emulsion suivant la revendication 2, caractérisée en ce que la paire de tampons est constituée par de l'acide citrique-citrate de sodium.

4. Emulsion suivant la revendication 1, caractérisée en ce qu'elle comprend en outre un ou plusieurs agents biologiquement actifs, solubles dans les graisses.

5. Emulsion suivant la revendication 4, caractérisée en ce que l'agent comprend une vitamine soluble dans le graisses.

6. Emulsion suivant la revendication 5, caractérisée en ce que la vitamine est une quantité nutritionnellement efficace des vitamines A, E, D ou K ou d'un mélange de celles-ci.

7. Emulsion suivant la revendication 5, caractérisée en ce qu'elle comprend en outre 0,25 à 2% en poids d'un ou de plusieurs aminoacides.

8. Emulsion suivant la revendication 7, caractérisée en ce que l'aminoacide est la L-lysine, la L-arginine ou un mélange de celles-ci.

9. Emulsion suivant la revendication 5, caractérisée en ce que l'huile est de l'huile de sésame, de l'huile de carthame, de l'huile de primevère, de l'huile de tournesol, de l'huile de poisson, de l'huile de graines de coton ou un mélange de celles-ci.

10. Emulsion suivant la revendication 9, caractérisée en ce que l'agent émulsifiant est efficace pour mettre en suspension les gouttelettes au moyen d'une pellicule multimoléculaire.

11. Emulsion suivant la revendication 5, caractérisée en ce que l'agent émulsifiant est de l'acacia, de la gélatine, de la gomme adragante, du chondre, de la pectine ou un mélange de ceux-ci.

12. Emulsion suivant la revendication 5, caractérisé en ce que l'agent stabilisant est un alcool polyatomique en $C_2$—$C_8$.

13. Emulsion suivant la revendication 12, caractérisée en ce que l'agent stabilisant est un mélange de glycérine et de sorbitol.

14. Emulsion suivant la revendication 4, caractérisée en ce que l'agent est une prostaglandine, un précurseur de prostaglandine d'acide gras insaturé à longue chaîne, du gossypol ou de la 5-azacytidine.

15. Emulsion suivant la revendication 5, caractérisée en ce qu'elle comprend en outre 0,025—0,5% en poids d'un antioxydant.

16. Emulsion vitaminée stable dans l'eau adaptée pour être facilement absorbée par les parois cellulaires et contenant des gouttelettes de phase huileuse d'un diamètre inférieur ou égal à 0,5 micromètre, comprenant:

(a) 30—80% en poids d'eau;

(b) une vitamine soluble dans l'huile qui est la vitamine A, D, E ou K, un dérivé pharmacologiquement stable de celles-ci ou un mélange de celles-ci, en une quantité thérapeutiquement efficace;

(c) un support d'huile végétale digestible pour la vitamine précitée comprenant des acides gras insaturés à longue chaîne compatibles avec la vitamine et en une quantité supérieure à celle-ci, dans lequel la quantité combinée de vitamine et de support est d'au moins 10% en poids inférieure à la quantité d'eau; et

(d) un émulsifiant digestible en une quantité correspondant à 5—40% du poids de l'émulsion.

17. Produit suivant la revendication 16, caractérisé en ce qu'un mélange de la vitamine et du support précités comprend des particules émulsifiées dans cette eau d'une taille de particules inférieure ou égale à 0,5 micromètre de diamètre.

18. Produit suivant la revendication 17, caractérisé en ce qu'il comprend un agent antimicrobien, un agent tampon et un stabilisant d'émulsion.

19. Produit suivant la revendication 18, caractérisé en ce qu'il comprend un ou plusieurs aminoacides.

20. Produit suivant la revendication 19, caractérisé en ce qu'il comprend un dérivé d'acide gras d'acide ascorbique.

21. Produit suivant la revendication 19, caractérisé en ce que l'huile est de l'huile de sésame ou de primevère;

l'émulsifiant est de la gomme arabique;

l'agent antimicrobien est un parabène et du benzoate d'alkyle;

l'agent tampon est un citrate avec de l'acide citrique;

le stabilisant d'émulsion est de la glycérine et du sorbitol; et

les aminoacides sont la lysine et l'arginine.

22. Produit suivant la revendication 21, caractérisé en ce qu'il comprend:

40—50% en poids d'eau;

10—30% en poids d'huile;

10—30% en poids d'émulsifiant;

0,05—1,0% en poids d'agent antimicrobien;

0,25—2,5% en poids d'agent tampon;

5—10% en poids de stabilisant d'émulsion; et

0,25—2% en poids d'aminoacides.

23. Procédé de formation d'une émulsion d'huile dans l'eau stable suivant l'une quelconque des revendications 1 à 15, qui comprend la formation comme phase huileuse d'un mélange d'un agent thérapeutique soluble dans l'huile, d'un support d'huile et d'un émulsifiant, la formation comme phase aqueuse d'un mélange d'eau, d'un stabilisant d'émulsion et d'un agent antimicrobien, la combinaison et le mélange de la phase huileuse et de la phase aqueuse et la soumission de ce mélange à une force de cisaillement élevée suffisante pour former des particules émulsifiées d'une taille inférieure ou égale à 0,5 micromètre de diamètre.

24. Procédé suivant la revendication 23, caractérisé en ce qu'il comprend:

(a) la formation comme phase huileuse homogène d'un mélange d'une ou de plusieurs huiles animales ou végétales contenant des graisses polyinsaturées, commestibles, d'une ou de plusieurs vitamines soluble dans les graisses, d'un ou de plusieurs aminoacides et d'un ou de plusieurs antioxydants;

(b) l'addition d'un agent émulsifiant au mélange agité de manière à former une pâte homogène;

(c) l'addition d'une solution aqueuse d'un ou de plusieurs agents stabilisants du type alcool polyatomique et d'un ou de plusieurs agents antimicrobiens à la pâte agitée;

(d) l'addition d'une solution aqueuse concentrée d'agents tampons organiques à la pâte avec une lente agitation pour former un mélange tamponné; et

(e) le passage du mélange tamponné dans un homogéniseur haute pression à $27{,}5 \times 10^6$—$41{,}2 \times 10^6$ Pa (4.000 à 6.000 livres par pouce carré), de manière à ce que les huiles et vitamines émulsifiées présentent une taille de gouttelettes inférieure ou égale à 0,5 micromètre de diamètre.